# EUROPÄISCHE PATENTSCHRIFT

(11) **EP 2 572 649 B1**
(45) Veröffentlichungstag und Bekanntmachung des Hinweises auf die Patenterteilung: **21.02.2018**
(21) Anmeldenummer: 12185261.0
(22) Anmeldetag: 20.09.2012
(51) Int. Cl.: A61B 17/04, A61B 17/42

(54) **Vorrichtung zur Hautdehnung**
Skin stretching device
Dispositif d'étirement de la peau

(30) Priorität: 23.09.2011 DE 102011114374
(43) Veröffentlichungstag der Anmeldung: 27.03.2013
(73) Patentinhaber: Karl Storz SE & Co. KG, 78532 Tuttlingen (DE)
(72) Erfinder: Hahn, Martin, 88637 Altheim (DE)
(74) Vertreter: Witte, Weller & Partner Patentanwälte mbB

(56) Entgegenhaltungen:
- DE-A1- 10 349 953
- FR-A1- 2 928 258
- RU-C2- 2 207 812
- US-A- 3 650 274
- US-A1- 2003 009 178
- US-A1- 2006 058 842

## Beschreibung

Die Erfindung betrifft eine Vorrichtung zur Hautdehnung, mit einem plattenförmigen Grundkörper, mit einem an dem Grundkörper angeordneten Spannelement, das um eine Drehachse drehbar ist, wodurch ein am Spannelement fixierter Zugfaden auf das Spannelement aufwickelbar und spannbar ist, mit einem Rastgesperre zum feststellen des drehbaren Spannelements und mit einer Sicherung zum Sichern des Rastgesperres gegen unbeabsichtigte lösen, wobei das Spannelement gegen die Kraft einer Feder in Richtung der Drehachse bewegbar ist, wodurch das Rastgesperre entrastbar ist, und wobei bei Freigabe des Spannelements dieses durch die Kraft der Feder wieder in die Raststellung rückkehrbar ist, und wobei die Sicherung durch einen am drehbaren Spannelement angeordneten Sicherungshebel bewirkbar ist, über den zugleich das Bewegen des Spannelementes sowohl in Richtung der Drehachse als auch um die Drehachse bewirkbar ist.

Eine derartige Vorrichtung zum Schließen von Wunden ist aus der US 2006/058842 A1 bekannt. Der Sicherungshebel ist dabei als ein runder Drehknopf ausgebildet der von einer Hand ergriffen werden kann und sowohl um die Drehachse als auch in Richtung der Drehachse bewegt werden kann.

Eine weitere Vorrichtung ist aus der DE 103 49 953 B4. Diese Vorrichtung dient zum Fixieren und Spannen zumindest eines Zugfadens für das Anlegen einer Neovagina. Unter dem Anlegen einer Neovagina wird eine Scheidenrekonstruktion verstanden, die beim Fehlen einer Scheidenanlage vorgenommen wird, das eine Fehlbildung des weiblichen Genitales ist, die beim Mayer-Rokitansky-Küster-Syndrom und bei testikulärer Feminisierung anzutreffen ist.

Das operative Prinzip zum Anlegen einer Neovagina besteht in einer Dehnung des Vaginalgrübchens. Über eine Kunststoffolive oder ein Steckphantom, das mit zwei Zugfäden verbunden ist, wird kontinuierlich Druck auf das Vaginalgrübchen ausgeübt. Dadurch wird dieser Hautbereich gedehnt und es bildet sich innerhalb von Tagen eine Neovagina. Die Zugfäden werden dabei auf das Spannelement der Vorrichtung zur Hautdehnung aufgewickelt. Im Rahmen des Verfahrens des Anlegens der Neovagina ist ein häufiges Nachspannen der Zugfäden erforderlich, da die Kunststoffolive bzw. das Steckphantom kontinuierlich Druck auf das Vaginalgrübchen ausüben und dieses dabei dehnen soll. Das Rastgesperre dient dazu, das Spannelement in einer bestimmten Drehstellung zum Spannen des Fadens zu halten, und zwar solange, bis sich die Haut entsprechend gedehnt hat. Danach wird das Spannelement erneut gedreht, um wieder zusätzliche Spannung in dem Faden aufzubauen. Das Rastgesperre weist ein seitlich neben dem Spannelement angeordnetes Betätigungselement auf, mit dem ein erstes Sperrelement zum Sperren mit einem gezahnten Rad in Eingriff gebracht werden kann, bzw. durch seitliches Wegbewegen von dem Spannelement die Rastsperre aufgehoben werden kann. Um gegen ein versehentliches Bewegen des Betätigungselements zu sichern, ist ein zweites noch weiter abseits des Spannelements angeordnetes verschiebbares zweites Betätigungselement angeordnet, das in den Bewegungsweg des ersten Sperrelements ein zweites Sperrelement einbringen kann, und zwar in dessen Verschiebeweg, so dass dann dieses erste Sperrelement gegenüber einem unbeabsichtigten Freigeben blockiert ist.

In der Dermatochirurgie, insbesondere in der plastischrekonstruktiven Chirurgie, ist ein Dehnen der Haut beidseits von Läsionen vor dem Entfernen der Läsion erforderlich. Beim Exzidieren oder Entfernen von Läsionen handelt es sich unter anderem um die Entfernung von Hauttumoren, von angeborenen Muttermalen oder auch von großflächigen Narben.

Dazu wird beidseits der Läsion ein plattenförmiger Grundkörper auf die Haut gelegt und diese beiden Grundkörper werden über subkutan geführte Zugfäden miteinander verbunden.

Zumindest auf einem der beiden Grundkörper befindet sich ein Spannelement, auf dem der oder die Zugfäden aufgewickelt und gespannt werden können. Dadurch bewegen sich die beiden plattenförmigen Grundkörper nach und nach aufeinander zu und wölben das zwischen den sich nach und nach aufeinander zu bewegenden plattenförmigen Grundkörpern vorhandene Gewebe, beispielsweise eine Narbe, auf. Der Hautbereich, der gedehnt werden soll, ist der jeweilige Hautbereich "hinter" den sich aufeinander zu bewegenden Platten.

Dabei ist, gegebenenfalls über einen Zeitraum von mehreren Wochen, ein permanentes Spannen und Nachspannen der Zugfäden erforderlich.

Dieses Nachspannen soll vom Patienten selbst einfach und sicher durchgeführt werden können, insbesondere deswegen, damit er nicht für jeden einzelnen Spannvorgang einen Arzt im Rahmen einer ambulanten Behandlung aufsuchen muss.

Die Vorrichtung soll insgesamt so aufgebaut sein, dass auch technisch wenig versierte Personen den Spannvorgang mit einfachen Handgriffen durchführen können.

Es ist daher Aufgabe der vorliegenden Erfindung, hier Abhilfe zu schaffen und eine Vorrichtung der eingangs genannten Art dahingehend weiter zu entwickeln, dass mit möglichst einfachem Aufbau ein solcher Spannvorgang einfach und sicher durchgeführt werden kann.

Erfindungsgemäß wird die Aufgabe dadurch gelöst, dass der Sicherungshebel umklappbar ist, und in einer ersten, vom Spannelement hochstehenden Stellung nicht sichert, und in einer zweiten, abgeklappten Stellung eine Bewegung des Spannelementes in Richtung der Drehachse blockiert, und dass der Sicherungshebel aus der zweiten abgeklappten Stellung in eine weitere Stellung verschiebbar ist, die ein hochklappen des Sicherungshebels hindert.

Diese Maßnahmen haben in konstruktiver Hinsicht den erheblichen Vorteil, dass alle für die Handhabung notwendigen Bauelemente direkt am Spannelement angeordnet sind. Bei der Handhabung als solcher muss nur ein einziges Bedienelement betätigt werden, nämlich der Sicherungshebel. Über diesen Sicherungshebel kann das Spannelement gegen die Kraft der Feder bewegt werden, wobei das Rastgesperre entrastet wird. Danach kann das Spannelement zum Spannen mittels des Sicherungshebels gedreht werden. Durch Freigabe oder durch Nachlassen der Kraft, die auf die Feder ausgeübt wird, drückt diese das Spannelement selbsttätig wieder in die Raststellung zurück. Dieser Sicherungshebel ist zugleich auch das Betätigungselement, mit dem die Sicherung gegen unbeabsichtigtes Bewegen des Spannelementes bewirkt wird.

Für den Patienten ist die Handhabung deswegen sehr einfach und sicher, da er nur ein einziges Element ergreifen und bedienen muss, nämlich den Sicherungshebel. Das Bewegen des Spannelements gegen die Kraft der Feder, also entweder ein Eindrücken oder ein Abziehen, ist ein einfacher Vorgang. Danach ist die freie Drehbarkeit gegeben. Gibt er das Spannelement frei, drückt die Feder das Rastgesperre automatisch sofort wieder in die sperrende Stellung. Zur Sicherung ist dann nur noch eine weitere Tätigkeit mit dem Sicherungshebel durchzuführen, um das Spannelement gegen unbeabsichtigtes Betätigen zu sichern.

Dies erleichtert die Handhabung für den Patienten erheblich und stellt eine Vorrichtung zur Verfügung, die kompakt baut, da alle Bauelemente am Spannelement selbst verwirklicht sind.

In einer weiteren Ausgestaltung sichert der Sicherungshebel in einer ersten, vom Spannelement hochstehenden Stellung nicht und ist in eine zweite, umgeklappte Stellung bringbar, in der er eine Bewegung des Spannelementes in Richtung der Drehachse blockiert.

Diese Maßnahme hat den Vorteil, dass in der hochstehenden Stellung der Sicherungshebel von der Bedienungsperson ergonomisch ergriffen werden kann, um das Spannelement zu bewegen, sprich gegen die Kraft der Feder längs der Drehachse zu bewegen und anschließend zum Spannen des Fadens zu drehen.

Durch Überbringen in die zweite abgeklappte Stellung steht zum einen der Sicherungshebel nicht weiter ab, somit auch nicht vom Körper des Patienten, so dass dieser darüber beispielsweise ein Kleidungsstück tragen kann, ohne dass zu erkennen ist, dass eine solche Vorrichtung zur Hautdehnung am Körper angebracht ist. In dieser umgeklappten Stellung erfüllt dann der Sicherungshebel seine Sicherungsfunktion, d.h. er blockiert jegliche Bewegung des Spannelementes.

In einer weiteren Ausgestaltung der Erfindung ist der Sicherungshebel um eine Hebelachse umklappbar, die senkrecht zur Drehachse des Spannelementes verläuft.

Diese Maßnahme hat den Vorteil, dass bei dieser Achsenausrichtung der Sicherungshebel in einer Stellung vom Spannelement hoch steht und somit einfach ergriffen werden kann. Durch Umklappen legt sich dann der Sicherungshebel auf das Spannelement und benötigt wenig Bauraum.

In einer weiteren Ausgestaltung der Erfindung ruht der Sicherungshebel über eine Nocke auf einem sich in der Drehachse erstreckenden Sicherungsstift, der in der ersten hochstehenden Stellung des Sicherungshebels eine Bewegung Spannelements längs der Drehachse erlaubt, in der zweiten, abgeklappten Stellung diese Bewegung blockiert.

Diese Maßnahme hat den Vorteil, dass beim Umklappen des Sicherungshebels über die Nocke der Sicherungsstift zwangsläufig in eine Stellung gebracht wird, die eine Bewegung des Spannelementes blockiert, somit die Sicherungsfunktion erfüllt ist.

Entsprechend der Erfindung ist der Sicherungshebel aus der zweiten abgeklappten Stellung in eine weitere Stellung verschiebbar, die ein Hochklappen des Sicherungshebels hindert.

Durch diese Maßnahme wird noch eine zusätzliche Sicherung erzielt.

Durch das Umklappen des Sicherungshebels ist zwar schon das Spannelement vor Bewegungen gesichert, allerdings kann der Sicherungshebel selbst, beispielsweise bei einer ungeschickten Körperbewegung oder bei Körperkontakt mit anderen Personen, versehentlich hochgeklappt werden, wodurch dann die Sicherung aufgehoben wäre.

Durch das Verschieben des Sicherungshebels in die weitere Stellung ist dieser nun selbst gegenüber Hochklappen blockiert, somit gesichert.

Um diese doppelte Sicherung aufzuheben, muss der Sicherungshebel zunächst aus der weiteren Stellung verschoben werden, und erst dann kann er hochgeklappt werden.

Dies ist unter dem Grundprinzip der Erfindung mit einem einzigen Bauelement mit einer einzigen Hand zu bewerkstelligen, so dass auch durch diese zusätzliche doppelte Sicherungsmaßnahme die einfache Handhabbarkeit nicht beeinträchtigt ist.

In einer weiteren Ausgestaltung der Erfindung weist die Nocke eine dem Sicherungsstift zugewandte abgerundete Nockenfläche auf, die ein axiales Verschieben des Sicherungsstiftes beim Umklappen des Sicherungshebels bewirkt.

Diese Maßnahme hat den Vorteil, dass die Umsetzung der Umklappbewegung des Sicherungshebels über die Nocke in eine lineare Verschiebung des Sicherungsstiftes sanft und zielgerecht erfolgt.

In einer weiteren Ausgestaltung der Erfindung weist der Sicherungshebel zumindest ein Langloch auf, in dem zumindest ein sich in der Hebelachse erstreckender Achsbolzen aufgenommen ist, wobei in der zweiten, abgeklappten Stellung des Sicherungshebels dieser um den Achsbolzen schwenkbar ist, wohingegen in der weiteren verschobenen Stellung des Sicherungshebels diese Schwenkbewegung blockiert ist.

Diese Maßnahme hat den Vorteil, dass durch mechanisch einfache und robuste Mittel diese zweite Stufe der Sicherung erzielt wird. Für die Handhabungsperson bedeutet das, das der Sicherungshebel aus dieser Stellung lediglich verschoben werden muss, wobei der Achsbolzen in dem Langloch läuft, und zwar bis er eine Stellung an einem Ende des Langloches erreicht hat, aus der der Sicherungshebel dann wieder hochgeklappt werden kann. Die Handhabungsperson spürt das, hat sie beispielsweise den Sicherungshebel nicht weit genug seitlich verschoben, ist er eben noch nicht hochklappbar, sondern erst wenn der Achsbolzen eine Endstellung des Langloches erreicht hat. Somit braucht die Handhabungsperson diese Verschiebbewegung nicht mit visueller Kontrolle durchzuführen, sondern kann das taktil feststellen, wenn der Achsbolzen an einem Ende des Langloches anschlägt.

In einer weiteren Ausgestaltung der Erfindung ist das Spannelement in einer Ausnehmung im Grundkörper aufgenommen, wobei die Drehachse des Spannelementes vom Grundkörper hoch steht, und das Spannelement ist in Richtung des Grundkörpers gegen die Kraft der Feder bewegbar.

Diese Maßnahme hat konstruktiv und handhabungsmäßig zahlreiche Vorteile. Zum einen steht das Spannelement nicht unnötig hoch und weit von dem Grundkörper ab, sondern ist zumindest teilweise in der Ausnehmung in den Grundkörper aufgenommen. Der Patient hat den plattenförmigen Grundkörper über die Zugfäden auf seinem Körper fixiert, beispielsweise im Bauchbereich oder an einem Oberschenkel. Für ihn ist es ergonomisch, bei hochgeklapptem Sicherungshebel das Spannelement gegen die Kraft der Feder weiter in den Grundkörper hinein zu drücken und dann die Drehung zu bewerkstelligen. Der an der Haut des Patienten über die Zugfäden fixierte plattenförmige Grundkörper gibt die nötige Widerstandskraft, um das Spannelement gegen die Kraft der Feder einzudrücken. In konstruktiver Sicht kann diese Feder dann zwischen dem Grundkörper und dem Spannelement angeordnet werden und diese wird beim Eindrücken des Spannelements in den Grundkörper entsprechend gespannt. Nach Freigabe drückt diese dann das Spannelement wieder so weit vom Grundkörper weg, dass das Rastgesperre sich in der rastenden Stellung befindet.

In einer weiteren Ausgestaltung der Erfindung weist das Rastgesperre zumindest eine in die Ausnehmung im Grundkörper vorstehende Raste auf, die in der Raststellung mit einem gezahnten Rad des Spannelementes im sperrenden Eingriff steht.

Diese Maßnahme hat den konstruktiven Vorteil, dass eine solche Raste relativ stabil ausgebildet und auch fest mit dem Grundkörper verbunden werden kann, indem diese beispielsweise in die Ausnehmung eingeschweißt wird. In der "Grundstellung" steht diese Raste mit einem gezahnten Rad des Spannelements in sperrendem Eingriff. Aus diesem sperrenden Eingriff wird das Spannelement ganz einfach dadurch gebracht, dass es gegen die Kraft der Feder in Richtung der Drehachse bewegt wird. Danach ist dann die Wirkung der Rastsperre aufgehoben und das Spannelement kann zum Aufwickeln und Spannen des Fadens gedreht werden. Wird das Spannelement freigegeben, drückt die Feder das gezahnte Rad des Spannelementes wieder in sperrenden Eingriff mit der Raste.

In einer weiteren Ausgestaltung der Erfindung sind die zumindest eine Raste und/oder die Zähne des gezahnten Rades an deren Flanken abgeschrägt.

Diese Maßnahme hat den Vorteil, dass diese automatische Einrastbewegung zu einem sicheren sperrenden Eingriff kommt und nicht ein Zahn des gezahnten Rades, wenn es genau auf die Raste trifft, eine weitere axiale Bewegung sperren würde. Durch die Anschrägungen verdreht sich das gezahnte Rad relativ zu der ortsfesten Raste zumindest so weit, dass die Raste zwischen zwei Zähnen eintreten kann.

In einer weiteren Ausgestaltung der Erfindung weist das Spannelement einen Spulenkörper auf, auf den der zumindest eine Zugfaden aufwickelbar ist, und der umklappbare Sicherungshebel ist auf dem dem Grundkörper abgewandten Ende des Spulenkörpers montiert.

Diese Maßnahme hat wiederum in konstruktiver Hinsicht den Vorteil, dass durch einfache und kompakt bauende Elemente sowohl das Aufwickeln und Spannen des Fadens durch den Spulenkörper, das Steuern des Spannelements zwischen den verschiedenen Stellungen durch den Sicherungshebel und letztendlich die Sicherung einfach zu bewerkstelligen ist.

In einer weiteren Ausgestaltung der Erfindung ist der Sicherungshebel als flächiges Element ausgebildet, das im abgeklappten Zustand auf dem oberen Ende des Spulenkörpers zum Liegen kommt.

Die Ausgestaltung als flächiges Element stellt der Handhabungsperson ein ausreichend großes flächiges Stück zur Verfügung, das er beispielsweise zwischen dem Daumen und dem Zeigefinger ergreifen kann. Nach dem Abklappen schmiegt sich das flächige Element auf die obere Seite des Spulenkörpers an und stellt kein unnötig hochstehendes Bauteil dar.

Ist eine derartige Vorrichtung beispielsweise im Bereich der Brust oder dem Bauch eines Patienten fixiert, kann er darüber Kleidungsstücke tragen, ohne dass die Gefahr besteht, dass diese sich mit abstehenden sperrigen Bauelementen verheddern. Zugleich ist auch nicht auf den ersten Blick zu erkennen, dass die Person eine solche Vorrichtung fixiert trägt, was der Patientencompliance sehr zuträglich ist.

In einer weiteren Ausgestaltung der Erfindung stehen vom flächigen Element zwei Laschen vor, in denen die Langlöcher ausgespart sind, und in den Langlöchern ist jeweils ein sich in der Hebelachse erstreckender Achsbolzen aufgenommen.

Diese Maßnahme hat den Vorteil, dass durch konstruktiv einfache Mittel eine feste Verbindung zwischen dem Sicherungshebel und dem Spannelement möglich ist, und zugleich die Beweglichkeit zum Hoch- und Abklappen des Sicherungshebels gegeben ist sowie, nach Umlegen, die seitliche Verschiebbarkeit.

In einer weiteren Ausgestaltung der Erfindung sind die Laschen so ausgebildet, dass diese bei abgeklapptem und seitlich verschobenem Sicherungshebel auf einer Seite der Hebelachse überstehen und am Spulenkörper anliegen, wodurch ein Hochklappen des Sicherungshebels gehindert ist.

Diese Maßnahme hat den erheblichen Vorteil, dass diese Laschen durch Anlage und Überstehen über die Hebelachse gegenüberliegend zum flächigen Element dafür sorgen, dass in diesem verschobenen Zustand der Sicherungshebel nicht mehr hochgeklappt werden kann.

In einer weiteren Ausgestaltung der Erfindung erstrecken sich die Laschen in der Ebene des flächigen Elements von diesem forterstreckend.

Diese Maßnahme hat den Vorteil, dass bei umgeklapptem und seitlich verschobenem Sicherungshebel dessen Bauelemente alle in einer Ebene liegen und sich eng an eine entsprechend ausgebildete Oberfläche des Spannelements anschmiegen können.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine Draufsicht auf eine Vorrichtung zur Hautdehnung, wobei die Führung von zwei Zugfäden dargestellt ist,
- Fig. 2: eine perspektivische Ansicht der Vorrichtung von Fig. 1,
- Fig. 3: einen Schnitt längs der Linie III-III in Fig. 1, allerdings ohne Zugfäden,
- Fig. 4: einen teilweisen Schnitt längs der Linie IV-IV in Fig. 1,
- Fig. 5: einen Schnitt längs der Linie V-V in Fig. 4 auf Höhe des Rastgesperres,
- Fig. 6: den Schnitt von Fig. 4 mit gegen die Kraft der Feder in den Grundkörper eingedrücktem Spannelement,
- Fig. 7: den entsprechenden Schnitt mit seitlich umgeklapptem Sicherungshebel,
- Fig. 8: einen der Fig. 7 entsprechenden Schnitt nach seitlichem Verschieben des umgeklappten Sicherungshebels,
- Fig. 9: eine perspektivische Ansicht der Vorrichtung von Fig. 1 mit seitlich abgeklapptem Sicherungshebel,
- Fig. 10: eine teilweise Seitenansicht auf die Vorrichtung von Fig. 9 längs des Pfeils 91,
- Fig. 11: eine der Fig. 9 vergleichbare perspektivische Darstellung nach seitlichem Verschieben des Sicherungshebels und
- Fig. 12: eine der Darstellung von Fig. 10 vergleichbare Darstellung nach seitlichem Verschieben des Sicherungshebels.

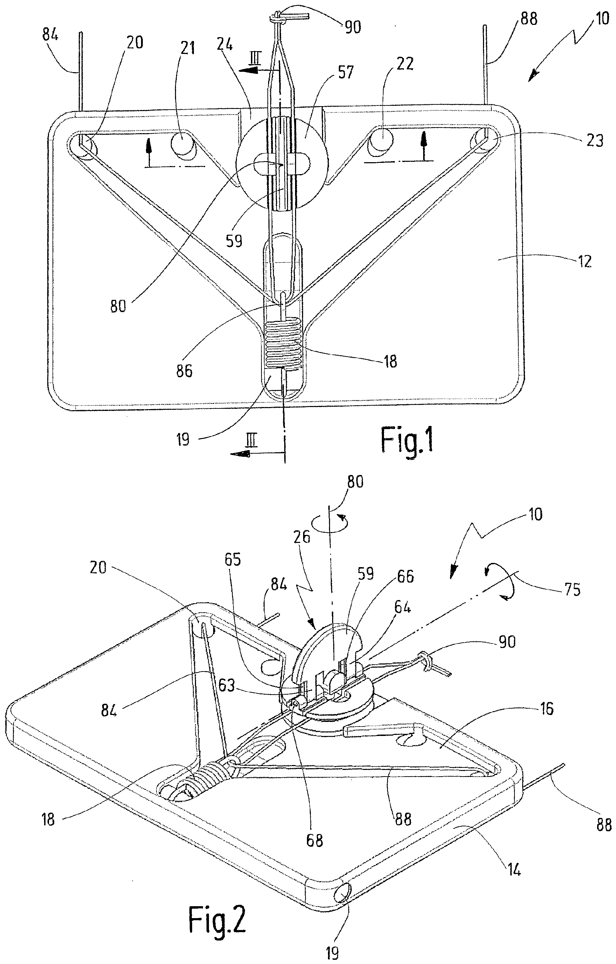

Ein in den Fig. 1 bis 12 dargestelltes Ausführungsbeispiel einer erfindungsgemäßen Vorrichtung zur Hautdehnung ist in ihrer Gesamtheit mit der Bezugsziffer 10 bezeichnet. Im Zusammenhang mit den Fig. 1 bis 6 sollen zunächst die konstruktiven Bauelemente der Vorrichtung 10 beschrieben werden. Die Vorrichtung 10 weist einen plattenförmigen Grundkörper 12 auf, der im dargestellten Ausführungsbeispiel aus einer rechteckförmigen ebenen Platte 14 aus Medizinstahl oder einem hautverträglichen Kunststoffmaterial besteht. Die Platte 14 hat beispielsweise das Ausmaß von 8x5 cm. Auf einer Seite der Platte 14 ist eine grob dreieckförmige Mulde 16 ausgespart. Im Bereich der Spitze der grob dreieckförmigen Mulde 16 ist eine Spannfeder 18 eingelegt, die über eine Stange 19 im plattenförmigen Grundkörper 12 verankert ist. Ein der Mulde 16 zugewandtes Ende der Spannfeder 18 weist eine Öse 86 auf, durch die, wie das später noch erläutert wird, Zugfäden 84 bzw. 88 geführt werden können.

Durch die Platte 14 hindurchgehend sind im Bereich der der Spannfeder 18 gegenüberliegenden längeren Rechteckseite vier Bohrungen 20, 21, 22, 23 vorhanden.

Diese Bohrungen 20 bis 23 dienen dazu, um durch die Platte 14 hindurch Zugfäden 84, 85 führen zu können.

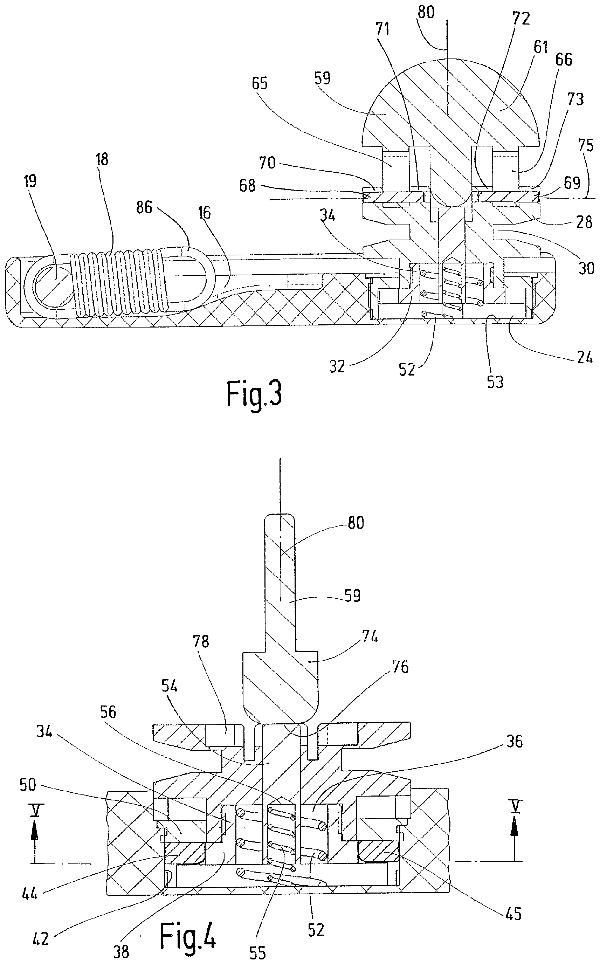

Im Bereich zwischen der Bohrungen 21 und 22 befindet sich eine Ausnehmung 24 in der Platte 14 (siehe auch Fig. 3), in die ein Spannelement 26 montiert ist.

Das Spannelement 26 weist einen Spulenkörper 28 mit etwa kreisförmigem Querschnitt auf, der eine umlaufende umfängliche Kerbe 30 aufweist. Am unteren Ende des Spulenkörpers 28 ist eine Hülse 32 eingeschraubt.

Die Hülse 32 weist einen hochstehenden zylindrischen Abschnitt 34 auf, der in eine Ausnehmung 36 im unteren Bereich des Spulenkörpers 28 hineinreicht und über entsprechende Innengewinde/Außengewinde fest mit dem Spulenkörper 28 verschraubt ist.

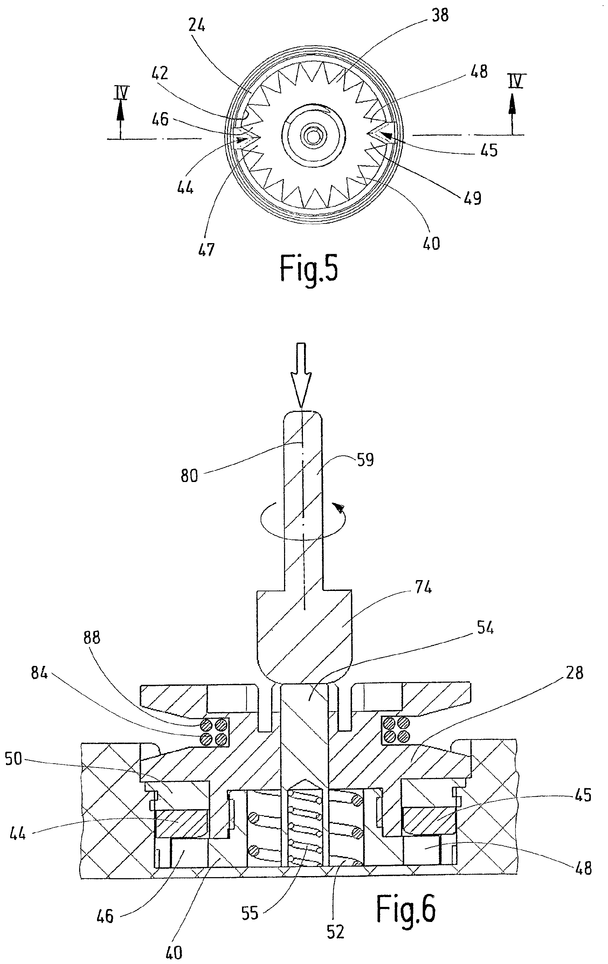

Am unteren Ende weist die Hülse 32 einen Ringflansch 38 auf, der als gezahntes Rad 40 ausgebildet ist, wie das insbesondere aus Fig. 5 ersichtlich ist.

Von der Innenwand 42 der Ausnehmung 36 stehen diametral gegenüberliegend zwei Rasten 44 und 45 vor, die mit der Innenwand 42 fest verschweißt sind.

Die Rasten 44 und 45 sind so ausgebildet und auf einer solchen Höhe angeordnet, dass diese mit Zähnen 46, 47, 48 und 49 des gezahnten Rades 40 in sperrendem Eingriff treten können. Das Spannelement 26 wird durch einen Haltering 50 unverlierbar in der Ausnehmung 36 gehalten.

Die Rasten 44 und 45 sowie das gezahnte Rad 40 mit dessen Zähnen 46 bis 49 bilden ein Rastgesperre 51. Das Rastgesperre 51 sperrt in der in Fig. 1 bis 5 dargestellten Position, die als "Grundstellung" des Spannelements 26 bezeichnet wird, ein Drehen des Spannelements 26 um dessen Drehachse 80. Im Innern der Hülse 32 ist eine erste Feder 52 aufgenommen, die sich einerseits auf dem Boden 53 der Ausnehmung 24 und gegenüberliegend am entsprechenden Boden in der Ausnehmung 36 im Spulenkörper 28 abstützt. Die erste Feder 52 ist so ausgestaltet, dass sie, falls keine Kraft auf sie einwirkt, das Spannelement 26 in dieser Grundstellung hält.

Mittig durch den Spulenkörper 28 durchgehend ist ein Sicherungsstift 54 vorgesehen, an dessen unterem Ende ein Sackloch 56 vorhanden ist, in dem eine zweite Feder 55 aufgenommen ist.

Dieser Sicherungsstift 54 ist im Bereich der Ausnehmung 36 im Spulenkörper 28 von der ersten Feder 52 umrundet. Die zweite Feder 55 stützt sich wiederum einerseits auf dem Boden 53 der Ausnehmung 24 bzw. dem Boden des Sackloches 56 ab. Auf dem oberen Ende 76 des Sicherungsstiftes 54 ruht eine Nocke 74 eines Sicherungshebels 59. Wie insbesondere aus den perspektivischen Darstellungen und aus der Schnittdarstellung von Fig. 3 ersichtlich, ist der Sicherungshebel 59 als ein flächiges Element 61 etwa in Form einer halbkreisförmigen Scheibe ausgebildet, von dem sich zwei Laschen 63 und 64 in dessen Erstreckungsebene weg erstrecken.

In jeder der Laschen 63 und 64 ist ein Langloch 65 bzw. 66 vorhanden.

Der Sicherungshebel 59 ist über zwei Achsbolzen 68 bzw. 69, die durch die Langlöcher 65 und 66 hindurchreichen, an der Oberseite des Spulenkörpers 28 montiert. Die Achsbolzen 68 und 69 sind dabei in entsprechenden beidseits der Laschen 63 und 64 angeordneten Lagerblöcken 70 und 71 bzw. 72 und 73 aufgenommen.

In den Fig. 1 bis 4 ist der Sicherungshebel 59 in seiner hochstehenden oder hochgeklappten Stellung dargestellt. Insbesondere aus den Schnittdarstellungen von Fig. 3 und 4 ist ersichtlich, dass sich die Unterseite des Spannelementes 26 in der dort ersichtlichen Grundstellung in einem Abstand vom Boden 53 der Ausnehmung 24 befindet.

Der gesamte Zusammenbau aus Spulenkörper 18, Hülse 32, Sicherungsstift 54 und Sicherungshebel 59 kann gegen die Kraft der ersten Feder 52 in Richtung des Bodens 53 der Ausnehmung 24 längs der Drehachse 80 verschoben werden.

Dazu kann der hochgeklappte Sicherungshebel 59 zwischen einem Daumen und einem Zeigefinger ergriffen werden und der Zusammenbau durch Handkraft eingedrückt werden.

Diese Situation ist in Fig. 6 dargestellt. Aus der Schnittdarstellung ist nun ersichtlich, dass das Spannelement 26 und somit auch das gezahnte Rad 40 so weit nach unten bewegt, also in die Ausnehmung 24 eingedrückt worden sind, dass die Zähne 46 und 48, man sieht in der Schnittdarstellung von Fig. 6 deren Flanken, aus dem sperrenden Eingriff mit den ortsfesten Rasten 44 und 45 getreten sind.

Nunmehr ist der Spulenkörper 28 frei drehbar und es können durch Drehen des Spannelementes 26 Zugfäden 84 und 88 auf den Spulenkörper 28 aufgewickelt werden.

Ein Beispiel einer Führung solcher Zugfäden 84 und 88 ist in Fig. 1 und 2 dargestellt. Ein erster Zugfaden 84 wird an die Unterseite der Platte 14 herangeführt und durch die Öffnung 20 in die Mulde 16 hineingeführt. Der erste Zugfaden 84 wird dann durch die Öse 86 der Spannfeder 18 geführt und anschließend zum Spulenkörper 28, und zwar so, dass dieser in dessen Kerbe 30 eingelegt ist.

Ein zweiter Zugfaden 88 ist entsprechend geführt, also an die Unterseite der Platte 14 heran, durch die Öffnung 23 hindurch, anschließend durch die Öse 86 der Spannfeder 18 und dann ebenfalls zur Kerbe 30 des Spulenkörpers 28.

Die beiden freien Enden der beiden Zugfäden 84 und 88 sind zu einem Knoten 90 verbunden.

Je nachdem welche Operationstechnik angewendet wird, bzw. welcher Hautdehnvorgang durchgeführt werden soll, sind die von der Platte 14 abgeführten Zugfäden 84 und 88 entsprechend verbunden.

Beim Anlegen einer Neovagina sind die Zugfäden, wie das in der eingangs erwähnten DE 103 49 953 B1 beschrieben ist, beispielsweise mit einer Kunststoffolive bzw. einem Steckphantom, das im Vaginalgrübchen eingelegt ist, verbunden.

Zum Fixieren und Spannen der Zugfäden wird der Sicherungshebel 59 in der hochstehenden oder hochgeklapptem Grundstellung ergriffen, das Spannelement 26 gegen die Kraft der Feder 52 eingedrückt und gedreht, wobei dann die Zugfäden 84 und 88 auf den Spulenkörper 28 aufgewickelt und je nach Drehzustand entsprechend gespannt werden.

Durch Freigabe des Sicherungshebels 59 oder durch Nachlassen der Eindrückkraft drückt die erste Feder 52 den Spulenkörper 28 in der Darstellung von Fig. 6 wieder nach oben, längs der Längsachse 80 in die in Fig. 4 dargestellte Grundstellung, in der dann wieder das Rastgesperre 51 wirkt.

Soll mit der Vorrichtung 10 eine Hautdehnung entsprechend der EP 2 279 701 A1 durchgeführt werden, sind die beiden Zugfäden 84 und 88 subkutan zu einer weiteren Platte geführt, die auf der gegenüberliegenden Seite der zu entfernenden Läsion angeordnet ist. Durch Spannen der Zugfäden werden dann diese beiden Platten aufeinander zu bewegt, die dazwischen liegende Läsion zu einem Wulst aufgetürmt, wobei dann die jeweils "hinter" den Platten liegenden Hautbereiche gedehnt werden.

Nur der Vollständigkeit halber soll erwähnt werden, dass beim Eindrücken auch gegen die Kraft der zweiten Feder 55, die im Sicherungsstift 54 aufgenommen ist, gedrückt werden muss. Die Hauptfunktion dieser zweiten Feder 55 ist aber, den Sicherungsstift 54 jeweils in Anlage mit der Nocke 74 des Sicherungshebels 59 zu halten.

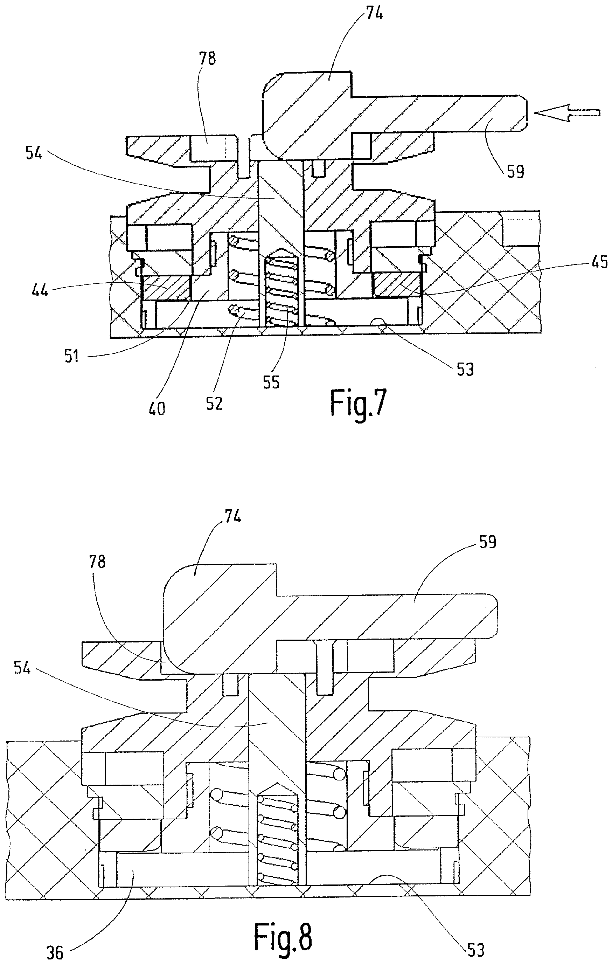

In Fig. 7 ist nun eine zweite Stellung des Sicherungshebels 59 dargestellt, nämlich die seitlich abgeklappte bzw. umgeklappte Position. Dieses Umklappen bzw. Abklappen des Sicherungshebels 59 erfolgt aus der in Fig. 4 dargestellten "Grundstellung". Aus dem Übergang von Fig. 4 zu Fig. 7 ist zu erkennen, dass beim Umklappen des Sicherungshebels 59 durch dessen Nocke 74 der Sicherungsstift 54 gegen die Kraft der zweiten Feder 55 bis zum Anschlag mit dem Boden 53 in die Ausnehmung 24 hineingedrückt wird. Hat der Sicherungsstift 54 diese in Fig. 7 dargestellte Position erreicht, ist es nun nicht mehr möglich, beispielsweise durch Drücken auf den abgeklappten Sicherungshebel 59 in Richtung der Drehachse 80 diesen Zusammenbau in die Ausnehmung 24 hinein zu bewegen, da diese Bewegung durch den Sicherungsstift 54 blockiert ist.

Aus der Schnittdarstellung von Fig. 7 ist ersichtlich, dass in diesem Zustand das Rastgesperre 51 in sperrendem Eingriff steht. Selbst wenn das äußere Ende des umgeklappten Sicherungshebels 59 ergriffen und in eine Drehbewegung versetzt werden soll, ist diese Bewegung durch das Rastgesperre 51 gesperrt.

Aus der Darstellung von Fig. 7 ist ersichtlich, dass der umgeklappte Sicherungshebel 59 relativ weit seitlich über den Spulenkörper 28 hinaus steht.

Es kann nun nicht ausgeschlossen werden, dass bei einer Person, an der eine derartige Vorrichtung 10 fixiert ist, durch unachtsame Handhabung oder durch Kleidungsstücke auf den Sicherungshebel 59 Kräfte dahingehend einwirken, die diesen hochklappen.

Daher ist vorgesehen, dass der Sicherungshebel aus der in Fig. 7 dargestellten abgeklappten Position noch zusätzlich seitlich verschoben werden kann, wobei dies durch den Übergang von Fig. 7 zu Fig. 8 dargestellt ist.

Auch in der in Fig. 8 dargestellten weiteren, seitlich verschobenen Stellung des umgeklappten Sicherungshebels 59 ruht dessen Nocke 74 auf dem Sicherungsstift 54, so dass nach wie vor die Sicherungsfunktion gegen unbeabsichtigtes Eindrücken des Spannelements 26 und damit unbeabsichtigtes Lösen der Rastsperre 51 sichergestellt ist. In der in Fig. 8 dargestellten Stellung ist der Sicherungshebel 59 allerdings nicht mehr hochklappbar.

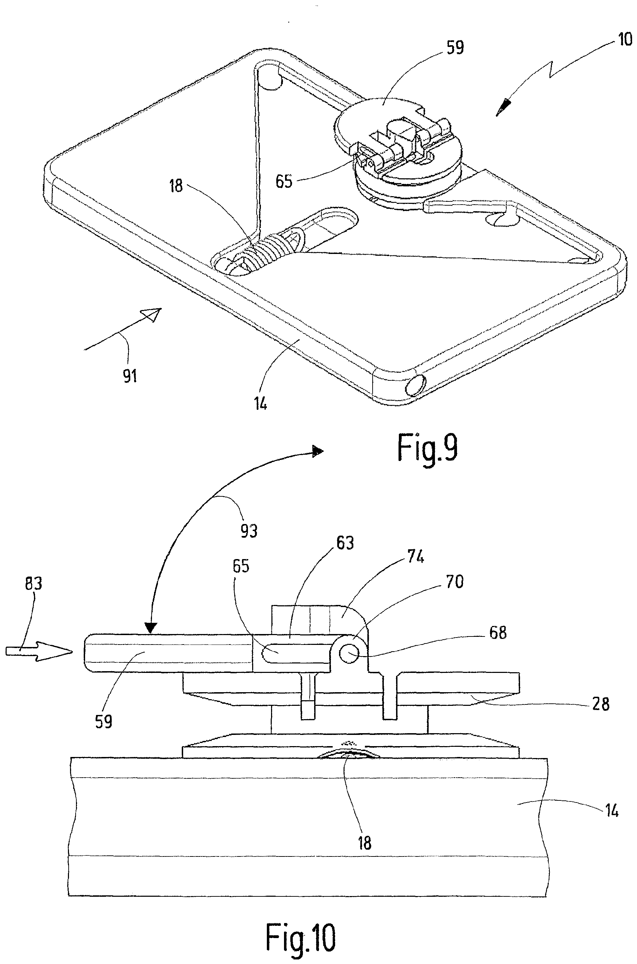

Dies soll nun in Zusammenhang mit den Fig. 9 bis 12 näher erläutert werden.

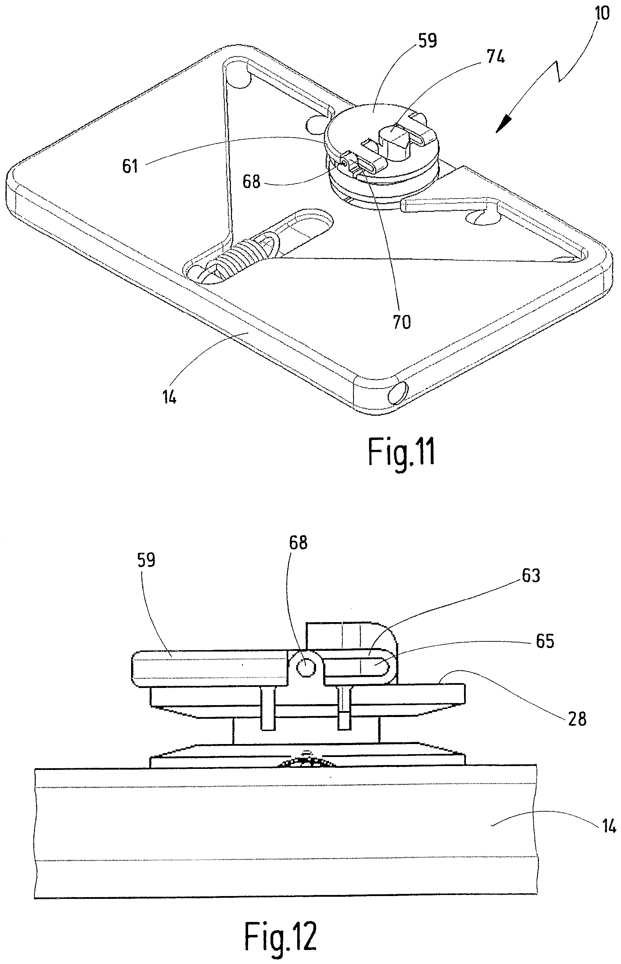

Wie bereits in Zusammenhang mit Fig. 2 und 3 erläutert, stehen vom Sicherungshebel 59 die beiden Laschen 63 und 64 vor, in deren Langlöchern 65 und 66 die Achsbolzen 68 und 69 eingeschoben sind. In der in Fig. 2 und 3 dargestellten Grundstellung befinden sich die Achsbolzen 68 und 69 am in dieser Darstellung "unteren" Ende der Langlöcher 65 und 66. In dieser Stellung ist, wie zuvor erwähnt, das Verschwenken, also das Hochstellen und das Abklappen des Sicherungshebels 59, möglich, wie das in Fig. 10 durch den Pfeil 93 angedeutet ist. Wird der Sicherungshebel 59 aus der in Fig. 9 und 10 dargestellten Position in die in Fig. 11 und 12 dargestellte Position seitlich verschoben, also längs des in Fig. 10 angedeuteten Pfeils 83, werden die Laschen 63 und 64 über die Achsbolzen 68 und 69 hinaus verschoben, bis die Achsbolzen 68 und 69 an dem gegenüberliegenden Ende der Langlöcher 65 und 66 anschlagen, wobei diese Position in Fig. 11 und 12 dargestellt ist.

Dabei liegen die über die Achsbolzen 68 und 69 hinausreichenden Bereiche der Laschen 63 und 64 auf der Oberseite des Spulenkörpers 28 an.

Somit blockieren diese überstehenden Laschen 63 uns 64 ein Hochstellen des Sicherungshebels 59 aus dieser weiteren, seitlich verschobenen Stellung des Sicherungshebels 59. Wie insbesondere aus Fig. 11 ersichtlich ist, liegt das etwa halbkreisförmige flächige Element 61 des Sicherungshebels 59 bündig auf der Oberseite des Spulenkörpers 28 an und überragt diese nicht mehr erheblich.

Damit ist nun die "doppelte" Sicherung bewerkstelligt. Der erste Sicherungsaspekt besteht darin, dass bei umgeklapptem Sicherungshebel 59, egal ob in der in Fig. 7 oder 8 dargestellten Schiebestellung, ein Eindrücken des Spannelementes 26 gesperrt ist, somit ein unbeabsichtigtes Bewegen und somit Freigeben der Rastsperre 51 angeschlossen ist.

In dem in Fig. 11 und 12 dargestellten seitlichen Verschiebezustand ist nun die zweite Stufe der Sicherung erzielt, nämlich der umgeklappte Sicherungshebel 59 selbst ist gegen Hochklappen blockiert.

Insbesondere aus den Darstellungen von Fig. 11 und 12 ist eindrucksvoll ersichtlich, dass die Vorrichtung keine sperrigen vorstehenden Bauelemente aufweist, die sich mit der Kleidung des Patienten verheddern können oder die Ansatzpunkte darstellen, über die das Spannelement 26 versehentlich betätigt werden könnte. Es ist ersichtlich, dass eine solche Vorrichtung 10, beispielsweise auf der Brust oder im Bauchbereich fixiert, kein erhebliches, insbesondere auch nicht von der Außenseite ersichtliches Hindernis darstellt.

Zum Betätigen der Vorrichtung muss der Patient ohnehin Zugriff zu dem Sicherungshebel 59 erhalten, so dass er notwendigerweise dann die Kleidungsstücke entfernen muss. Durch seitliches Verschieben des Sicherungshebels 59 aus der Stellung von Fig. 12 in Fig. 10 schafft er die Grundvoraussetzung, damit der Sicherungshebel hochgeklappt werden kann.

Diese Position erkennt er zum einen daraus, dass nunmehr der Sicherungshebel 59 seitlich über den Spulenkörper übersteht bzw. er bemerkt das dadurch, dass er diesen nicht mehr weiter in dieser Richtung verschieben kann, da dies durch den Anschlag der Achsbolzen 68 und 69 in den Langlöchern 65 und 66 blockiert ist. Nunmehr kann er, wie das in Fig. 10 dargestellt ist, den Sicherungshebel 59 hochklappen.

Durch Eindrücken und Drehen, wie das in Fig. 6 dargestellt ist, kann zunächst die Rastsperre 51 gelöst und der Spulenkörper 28 gedreht werden, wodurch dann die Zugfäden 84 und 88 nach und nach aufgewickelt und entsprechend gespannt werden. Hat er die ausreichende Zugspannung erzielt, er spürt den Zugschmerz der Zugfäden, gibt er das Spannelement 26 frei bzw. lässt die Eindruckkraft nach, so dass das Spannelement durch die Kraft der ersten Feder 52 wieder nach oben in die sperrende Stellung gedrückt wird. Danach klappt er den Sicherungshebel 59 wieder um, verschiebt diesen seitlich und wartet bis zum nächsten Spannvorgang.

## Patentansprüche

1. Vorrichtung zur Hautdehnung, mit einem plattenförmigen Grundkörper (12), mit einem an dem Grundkörper (12) angeordneten Spannelement (26), das um eine Drehachse (80) drehbar ist, wodurch ein am Spannelement (26) fixierter Zugfaden (84, 88) auf das Spannelement (26) aufwickelbar und spannbar ist, mit einem Rastgesperre (51) zum Feststellen des drehbaren Spannelementes (26) und mit einer Sicherung zum Sichern des Rastgesperres (51) gegen unbeabsichtigtes Lösen, wobei das Spannelement (26) gegen die Kraft einer Feder (52) in Richtung der Drehachse (80) bewegbar ist, wodurch das Rastgesperre (51) entrastbar ist, und wobei bei Freigabe des Spannelementes (26) dieses durch die Kraft der Feder (52) wieder in die Raststellung rückkehrbar ist, und wobei die Sicherung durch einen am drehbaren Spannelement (26) angeordneten Sicherungshebel (59) bewirkbar ist, über den zugleich das Bewegen des Spannelementes (26) sowohl in Richtung der Drehachse (80) als auch um die Drehachse (80) bewirkbar ist **dadurch gekennzeichnet, dass** der Sicherungshebel (59) umklappbar ist, und in einer ersten, vom Spannelement (26) hochstehenden Stellung nicht sichert, und in einer zweiten, abgeklappten Stellung eine Bewegung des Spannelementes (26) in Richtung der Drehachse (80) blockiert, und dass der Sicherungshebel (59) aus der zweiten abgeklappten Stellung in eine weitere Stellung verschiebbar ist, die ein Hochklappen des Sicherungshebels (59) hindert.

2. Vorrichtung nach Anspruch 1, **dadurch gekennzeichnet, dass** der Sicherungshebel (59) um eine Hebelachse (75) umklappbar ist, die senkrecht zur Drehachse (80) des Spannelementes (26) verläuft.

3. Vorrichtung nach Anspruch 1 oder 2, **dadurch gekennzeichnet, dass** der Sicherungshebel (59) über eine Nocke (74) auf einem sich in der Drehachse (80) erstreckenden Sicherungsstift (54) ruht, der in der ersten, hochgeklappten Stellung des Sicherungshebels (59) eine Bewegung des Spannelements (26) längs der Drehachse (80) erlaubt, in der zweiten, abgeklappten Stellung diese Bewegung blockiert.

4. Vorrichtung nach Anspruch 3, **dadurch gekennzeichnet, dass** die Nocke (74) eine dem Sicherungsstift (54) zugewandte abgerundete Nockenfläche aufweist, die ein axiales Verschieben des Sicherungsstiftes (54) beim Umklappen des Sicherungshebels (54) bewirkt.

5. Vorrichtung nach einem der Ansprüche 1 bis 4, **dadurch gekennzeichnet, dass** der Sicherungshebel (54) zumindest ein Langloch (55, 56) aufweist, in dem zumindest ein sich in der Hebelachse (75) erstreckender Achsbolzen (68, 69) aufgenommen ist, wobei in der zweiten abgeklappten Stellung des Sicherungshebels (59) dieser um diesen Achsbolzen (68, 69) schwenkbar ist, in der weiteren seitlich verschobenen Stellung des Sicherungshebels (54) diese Schwenkbewegung jedoch gehindert ist.

6. Vorrichtung nach einem der Ansprüche 1 bis 5, **dadurch gekennzeichnet, dass** das Spannelement (26) in einer Ausnehmung (24) im Grundkörper (12) aufgenommen ist, dass ferner die Drehachse (80) vom Grundkörper (12) hoch steht, und dass das Spannelement (26) in Richtung des Grundkörpers (12) gegen die Kraft der Feder (52) bewegbar ist.

7. Vorrichtung nach Anspruch 6, **dadurch gekennzeichnet, dass** das Rastgesperre (51) zumindest eine in die Ausnehmung (24) im Grundkörper (12) vorstehende Raste (44, 46) aufweist, die in der Raststellung mit einem gezahnten Rad (40) des Spannelements (26) in sperrendem Eingriff steht.

8. Vorrichtung nach Anspruch 7, **dadurch gekennzeichnet, dass** die zumindest eine Raste (44, 46) und/oder Zähne (46 bis 49) des gezahnten Rades (40) an deren Flanken abgeschrägt sind.

9. Vorrichtung nach einem der Ansprüche 1 bis 8, **dadurch gekennzeichnet, dass** das Spannelement (26) einen Spulenkörper (28) aufweist, auf den der zumindest eine Zugfaden (84, 88) aufwickelbar ist, und dass der umklappbare Sicherungshebel (59) auf dem dem Grundkörper (12) abgewandten Ende (57) des Spulenkörpers (28) montiert ist.

10. Vorrichtung nach Anspruch 9, **dadurch gekennzeichnet, dass** der Sicherungshebel (59) als flächiges Element (61) ausgebildet ist, das im abgeklappten Zustand auf dem Ende (57) des Spulenkörpers (28) zum Liegen kommt.

11. Vorrichtung nach Anspruch 10, **dadurch gekennzeichnet, dass** vom flächigen Element (61) zwei Laschen (63, 64) vorstehen, in denen Langlöcher (65, 66) ausgespart sind, und dass in den Langlöchern (65, 66) jeweils ein sich in der Hebelachse (75) erstreckender Achsbolzen (68, 69) aufgenommen ist.

12. Vorrichtung nach Anspruch 11, **dadurch gekennzeichnet, dass** die Laschen (63, 64) so ausgebildet sind, dass diese bei abgeklapptem und seitlich verschobenem Sicherungshebel (59) auf einer Seite der Hebelachse (75) überstehen und am Spulenkörper (28) anliegen, wodurch ein Hochklappen des Sicherungshebels (59) gehindert ist.

13. Vorrichtung nach Anspruch 11 oder 12, **dadurch gekennzeichnet, dass** sich die Laschen (63, 64) in der Ebene des flächigen Elements (61) von diesem forterstrecken.

## Claims

1. Device for stretching skin, comprising a plate-shaped main body (12), comprising a tensioning element (26) arranged on the main body (12), said tensioning element being rotatable about an axis of rotation (80), as a result of which a pulling thread (84, 88) affixed on the tensioning element (26) can be wound up on the tensioning element (26) and tensioned, comprising a latching lock (51) for fixing the rotatable tensioning element (26) and comprising securing means for securing the latching lock (51) against inadvertent release, wherein the tensioning element (26) can be moved in the direction of the axis of rotation (80) against the force from a spring (52), as a result of which the latching lock (51) can be disengaged, wherein when the tensioning element (26) is released the latter can be returned to the latching position as a result of the force from the spring (52), and wherein the securing means can be effected by a securing lever (59) arranged on the rotatable tensioning element (26), by means of which securing lever the movement of the tensioning element (26) both in the direction of the axis of rotation (80) and about the axis of rotation (80) can be effected at the same time, **characterized in that** the securing lever (59) is pivotable and does not secure in a first position, raised from the tensioning element (26), and blocks a movement of the tensioning element (26) in the direction of the axis of rotation (80) in a second, pivoted-down position, and **in that** the securing lever (59) can be displaced, from the second, pivoted-down position, into a further position which prevents the securing lever (59) from pivoting upwards.

2. Device according to Claim 1, **characterized in that** the securing lever (59) can be pivoted about a lever axis (75) which runs perpendicularly to the axis of rotation (80) of the tensioning element (26).

3. Device according to Claim 1 or 2, **characterized in that** the securing lever (59) rests, via a cam (74), on a securing pin (54) extending along the axis of rotation (80), said securing pin allowing a movement of the tensioning element (26) along the axis of rotation (80) in the first, pivoted-upwards position of the securing lever (59), and blocking said movement in the second, pivoted-down position.

4. Device according to Claim 3, **characterized in that** the cam (74) comprises a rounded cam surface facing the securing pin (54), said cam surface effecting an axial displacement of the securing pin (54) when the securing lever (54) is pivoted over.

5. Device according to one of Claims 1 to 4, **characterized in that** the securing lever (54) comprises at least one slot (55, 56), in which at least one axle pin (68, 69) extending along the lever axis (75) is held, wherein, in the second, pivoted-down position of the securing lever (59), the latter can be pivoted about this axle pin (68, 69), wherein, however, this pivot movement is prevented in the further laterally displaced position of the securing lever (54).

6. Device according to one of Claims 1 to 5, **characterized in that** the tensioning element (26) is held in a cut-out (24) in the main body (12), **in that**, additionally, the axis of rotation (80) is raised from the main body (12), and **in that** the tensioning element (26) is movable in the direction of the main body (12) against the force from the spring (52).

7. Device according to Claim 6, **characterized in that** the latching lock (51) comprises at least one latch (44, 46) protruding into the cut-out (24) in the main body (12), which latch, in the latching position, is in blocking engagement with a toothed wheel (40) of the tensioning element (26).

8. Device according to Claim 7, **characterized in that** the at least one latch (44, 46) and/or teeth (46 to 49) of the toothed wheel (40) are chamfered at the flanks thereof.

9. Device according to one of Claims 1 to 8, **characterized in that** the tensioning element (26) comprises a reel body (28), onto which the at least one pulling thread (84, 88) can be wound, and **in that** the pivotable securing lever (59) is assembled on the end (57) of the reel body (28) facing away from the main body (12).

10. Device according to Claim 9, **characterized in that** the securing lever (59) is embodied as an areal element (61), which comes to rest on the end (57) of the reel body (28) in the pivoted-down state.

11. Device according to Claim 10, **characterized in that** from the areal element (61) two flaps (63, 64) protrude, in which flaps slots (65, 66) have been cut out, and **in that** an axle pin (68, 69) extending along the lever axis (75) is respectively held in the slots (65, 66).

12. Device according to Claim 11, **characterized in that** the flaps (63, 64) are formed such that these project on one side of the lever axis (75) and rest against the reel body (28) in the case of a pivoted-down and laterally displaced securing lever (59), as a result of which the securing lever (59) is prevented from being pivoted upwards.

13. Device according to Claim 11 or 12, **characterized in that** the flaps (63, 64) extend away from the areal element (61) in the plane of the latter.

## Revendications

1. Dispositif d'étirement de la peau, avec un corps de base en forme de plaque (12), avec un élément de serrage (26) disposé sur le corps de base (12), qui peut tourner autour d'un axe de rotation (80), grâce auquel un fil de traction (84, 88) fixé à l'élément de serrage (26) peut être enroulé et serré sur l'élément de serrage (26), avec un dispositif d'encliquetage à crans (51) pour immobiliser l'élément de serrage tournant (26) et avec un dispositif de sécurité pour bloquer le dispositif d'encliquetage (51) contre une libération imprévue, dans lequel l'élément de serrage (26) est déplaçable dans la direction de l'axe de rotation (80) contre la force d'un ressort (52), ce qui permet de libérer le dispositif d'encliquetage (51), et dans lequel lors de la libération de l'élément de serrage (26) celui-ci peut de nouveau retourner dans la position d'encliquetage par la force du ressort (52), et dans lequel le dispositif de sécurité peut être actionné par un levier de sécurité (59) disposé sur l'élément de serrage tournant (26), au moyen duquel le mouvement de l'élément de serrage (26) aussi bien dans la direction de l'axe de rotation (80) qu'autour de l'axe de rotation (80) peut être provoqué simultanément, **caractérisé en ce que** le levier de sécurité (59) est rabattable, et dans une première position dressée sur l'élément de serrage (26) ne bloque pas, et dans une deuxième position rabattue bloque un mouvement de l'élément de serrage (26) dans la direction de l'axe de rotation (80), et **en ce que** le levier de sécurité (59) est déplaçable de la deuxième position rabattue à une autre position, qui empêche un relèvement du levier de sécurité (59).

2. Dispositif selon la revendication 1, **caractérisé en ce que** le levier de sécurité (59) est rabattable autour un axe de levier (75), qui est perpendiculaire à l'axe de rotation (80) de l'élément de serrage (26).

3. Dispositif selon la revendication 1 ou 2, **caractérisé en ce que** le levier de sécurité (59) repose par une came (74) sur une goupille de sécurité (54) s'étendant suivant l'axe de rotation (80), qui dans la première position relevée du levier de sécurité (59) permet un mouvement de l'élément de serrage (26) le long de l'axe de rotation (80) et dans la deuxième position rabattue bloque ce mouvement.

4. Dispositif selon la revendication 3, **caractérisé en ce que** la came (74) présente une face de came arrondie tournée vers la goupille de sécurité (54), qui provoque un déplacement axial de la goupille de sécurité (54) lors du rabattement du levier de sécurité (54).

5. Dispositif selon l'une quelconque des revendications 1 à 4, **caractérisé en ce que** le levier de sécurité (54) présente au moins un trou allongé (55, 56), dans lequel est logé au moins un pivot axial (68, 69) s'étendant suivant l'axe de levier (75), dans lequel dans la deuxième position rabattue du levier de sécurité (59) celui-ci peut pivoter autour de ces pivots axiaux (68, 69), et dans l'autre position déplacée latéralement du levier de sécurité (54) ce mouvement de pivotement est cependant empêché.

6. Dispositif selon l'une quelconque des revendications 1 à 5, **caractérisé en ce que** l'élément de serrage (26) est logé dans un évidement (24) dans le corps de base (12), **en ce qu'**en outre l'axe de rotation (80) est dressé sur le corps de base (12) et **en ce que** l'élément de serrage (26) est déplaçable en direction du corps de base (12) contre la force du ressort (52).

7. Dispositif selon la revendication 6, **caractérisé en ce que** le dispositif d'encliquetage à crans (51) présente au moins un cliquet (44, 46) saillant dans l'évidement (24) dans le corps de base (12), qui dans la position d'encliquetage est en prise de blocage avec une roue dentée (40) de l'élément de serrage (26).

8. Dispositif selon la revendication 7, **caractérisé en ce que** ledit au moins un cliquet (44, 46) et/ou les dents (46 à 49) de la roue dentée (40) ont des flancs inclinés.

9. Dispositif selon l'une quelconque des revendications 1 à 8, **caractérisé en ce que** l'élément de serrage (26) présente un corps de bobine (28), sur lequel ledit au moins un fil de traction (84, 88) peut être enroulé, et **en ce que** le levier de sécurité rabattable (59) est monté sur l'extrémité (57) du corps de bobine (28) située à l'opposé du corps de base (12).

10. Dispositif selon la revendication 9, **caractérisé en ce que** le levier de sécurité (59) est réalisé sous forme d'élément plat (61), qui dans l'état rabattu vient se placer sur l'extrémité (57) du corps de bobine (28).

11. Dispositif selon la revendication 10, **caractérisé en ce que** deux pattes (63, 64) sont en saillie sur l'élément plat (61), dans lesquelles des trous allongés (65, 66) sont pratiqués, et **en ce qu'**un pivot axial (68, 69) s'étendant suivant l'axe de levier (75) est logé dans chacun des trous allongés (65, 66).

12. Dispositif selon la revendication 11, **caractérisé en ce que** les pattes (63, 64) sont réalisées de telle manière que, lorsque le levier de sécurité (59) est rabattu et déplacé latéralement, elles soient saillantes sur un côté de l'axe de levier (75) et s'appliquent sur le corps de bobine (28), ce qui empêche un relèvement du levier de sécurité (59).

13. Dispositif selon la revendication 11 ou 12, **caractérisé en ce que** les pattes (63, 64) se prolongent dans le plan de l'élément plat (61) à partir de celui-ci.
